# EUROPEAN PATENT SPECIFICATION

(11) **EP 2 589 692 B1**
(45) Date of publication and mention of the grant of the patent: **11.07.2018**
(21) Application number: 11800855.6
(22) Date of filing: 28.06.2011
(51) Int. Cl.: D04H 1/42, A61K 8/02, A61K 8/73, A61K 9/70, A61K 47/36, A61Q 19/00, B32B 5/26, D04H 1/72, A61K 9/00, A61K 8/67, A61K 8/81, A61Q 19/02

(54) **NANOFIBRE LAMINATE SHEET**
NANOFASER-LAMINATFOLIE
FEUILLE STRATIFIÉE À BASE DE NANOFIBRE

(30) Priority: 23.06.2011 JP 2011139753; 29.06.2010 JP 2010148304
(43) Date of publication of application: 08.05.2013
(73) Proprietor: Kao Corporation, Chuo-Ku Tokyo 103-8210 (JP)
(72) Inventor: TOJO, Takehiko, Haga-gun Tochigi 321-3497 (JP); ISHIKAWA, Masataka, Haga-gun Tochigi 321-3497 (JP)
(74) Representative: Vossius & Partner Patentanwälte Rechtsanwälte mbB
(86) International application number: PCT/JP2011/064811
(87) International publication number: WO 2012/002391

(56) References cited:
- EP-A1- 2 103 424
- EP-A1- 2 394 670
- WO-A2-2009/120365
- JP-A- 2008 179 629
- JP-A- 2008 514 341
- JP-B2- 3 944 526

## Description

### Technical Field

The present invention relates to a laminate sheet composed of a nanofiber layer and a water soluble base layer.

### Background Art

Nanofibers are applied to the fields demanding optical characteristics such as high transparency, where the nano-size effect of nanofibers are taken advantage of. For example, nanofibers with a diameter equal to or below the wavelength of visible light provide transparent fabric. By the use of nanofibers the diameter of which is equal to the wavelength of visible light, structural color may be exhibited. Nanofibers have also been studied for their applicability to the fields demanding superabsorbent characteristics or high surface activity, where the high specific surface area effect of nanofibers is taken advantage of, and to the fields demanding mechanical characteristics such as tensile strength and electrical characteristics such as high conductivity, where the supramolecular arrangement effect of nanofibers is made use of. Nanofibers having such characteristics have been used in the form of, for example, not only single fibers but aggregates (i.e., fabrics) or composites.

Applications of nanofibers that have been proposed include a cosmetic sheet comprising a network structure made of a water soluble polymer nanofiber and a cosmetic or a cosmetic component, such as ascorbic acid, held in the network structure as disclosed in patent literature 1 below. Patent literature 1 alleges that the cosmetic sheet has not only improved adhesion or comfort to the user's face, hand, or leg but also storage stability. Patent literature 2 below describes that a functional component, such as an emulsifying component, a stabilizing component, a bactericidal component, a moisturizing component, and so on, may be added to a nanofiber sheet.

### Citation List

### Patent Literature

Patent literature 1: JP 2008-179629A
Patent literature 2: WO 2009/031620A1

### Summary of Invention

### Technical Problem

A nanofiber prepared from a water soluble polymer exhibits higher water solubility than in its bulk form on account of the large specific surface area and, therefore, easily dissolves with water, e.g., of perspiration just upon being touched with a finger. It is likely that the nanofiber sheet dissolves or gets a hole in it before it is attached to an object. That is, the water soluble polymer-based nanofiber sheet is less than easy to handle.

### Solution to Problem

The invention settles the above discussed problem by providing a nanofiber laminate sheet composed of a layer of a nanofiber (hereinafter a nanofiber layer) containing a cosmetic component or a medicinal component and a water soluble base layer located on at least one side of the nanofiber layer. The water soluble base layer is less water-soluble than the nanofiber layer.

The invention includes the following subject matter.
[1] A nanofiber laminate sheet comprising a nanofiber layer containing a cosmetic component or a medicinal component and a water soluble base layer located on at least one side of the nanofiber layer, the water soluble base layer being less water-soluble than the nanofiber layer.
[2] The nanofiber laminate sheet set forth in [1] above, wherein the dissolving time ratio of water soluble base layer to nanofiber layer is 1.1 or greater, the dissolving time being measured by the method I described later.
[3] The nanofiber laminate sheet set forth in [2] above, wherein the dissolving time ratio of water soluble base layer to nanofiber layer is 1.1 to 50, the dissolving time being measured by the method I described later.
[4] The nanofiber laminate sheet set forth in [1], wherein the dissolving time ratio of water soluble base layer to nanofiber layer is 1.1 or greater, the dissolving time being measured by the method II described later.
[5] The nanofiber laminate sheet set forth in [4], wherein the dissolving time ratio of water soluble base layer to nanofiber layer is 1.1 to 200, the dissolving time being measured by the method II described later.
[6] The nanofiber laminate sheet set forth in any one of [1] to [5], wherein the water soluble base layer and the nanofiber comprise the same material, the water soluble base layer has a smaller specific surface area than the nanofiber layer, and the water soluble base layer comprises a poreless film, a porous film, or a mesh.
[7] Te nanofiber laminate sheet set forth in any one of [1] to [6], wherein the nanofiber comprises a water soluble polymer, and the water soluble polymer is one of, or a combination of two or more of, naturally occurring polymers, such as mucopolysaccharides, e.g., pullulan, hyaluronic acid, chondroitin sulfate, poly-γ-glutamic acid, modified corn starch, β-glucan, gluco-oligosaccharides, heparin, and keratosulfate, cellulose, pectin, xylan, lignin, glucomannan, galacturonic acid, psyllium seed gum, tamarind seed gum, gum arabic, tragacanth gum, soybean water-soluble polysaccharides, alginic acid, carrageenan, laminaran, agar (agarose), fucoidan, methyl cellulose, hydroxypropyl cellulose, and hydroxypropylmethyl cellulose; partially saponified polyvinyl alcohol (usable in the absence of a crosslinking agent), low-saponified polyvinyl alcohol, polyvinylpyrrolidone (PVP), polyethylene oxide, and sodium polyacrylate, preferably, pullulan, partially saponified polyvinyl alcohol, low-saponified polyvinyl alcohol, polyvinylpyrrolidone, or polyethylene oxide.
[8] The nanofiber laminate sheet set forth in [7], wherein pullulan is used as a nanofiber.
[9] The nanofiber laminate sheet set forth in any one of [1] to [8], wherein the water soluble base layer is a film prepared from a polysaccharide material, such as oblate and pullulan, polyvinyl alcohol, agar, or carrageenan.
[10] The nanofiber laminate sheet set forth in [9], wherein pullulan is used as the water soluble base layer.
[11] The nanofiber laminate sheet set forth in [9], wherein the water soluble base layer is a oblate film.
[12] The nanofiber laminate sheet set forth in [9], wherein the water soluble base layer is a polyvinyl alcohol film.
[13] The nanofiber laminate sheet set forth in any one of [1] to [12], wherein the cosmetic component or a medicinal component is a blood circulation improving agent selected from acid mucopolysaccharides, chamomile, buckeye, ginkgo, witch hazel extract, vitamin E, nicotinic acid derivatives, and alkaloid compounds; an edema reducing agent selected from buckeye, flavone derivatives, naphthalenesulfonic acid derivatives, anthocyanin, vitamin P, calendula, concholytic acid, silanol, terminalia, visnaga, and majus; a slimming agent selected from aminophylline, tea extract, caffeine, xanthine derivatives, inositol, dextransulfuric acid derivatives, buckeye, aescin, anthocyanidin, organoiodine compounds, hypericum, Filipendula multijuga, field horsetail, rosemary, ginseng, ivy, thiomucase, and hyaluronidase; a painkiller selected from indometacin, diclofenac, dl-camphor, flurbiprofen, ketoprofen, capsicum extract, piroxicam, felbinac, methyl salicylate, and glycol salicylate; a moisturizing agent selected from polyols, ceramids, and collagens; a peeling gent including a protease; a depilatory agent including calcium thioglycolate; an autonomic regulatory agent including γ-oryzanol; an extract of Japanese elk horn ceder (Thujopsis dolabrata), balloon flower root, eucalyptus, birch, ginger, yuzu (Citrus junos), etc.; tranexamic acid, allantoin, stearyl glycyrrhetinate, niacinamide, 1-menthol, and avitamin, such as vitamin C.
[14] The nanofiber laminate sheet set forth in any one of [1] and [13], wherein the content of the cosmetic component or the medicinal component in the nanofiber is in the range of from 0.01% to 70% by mass.
[15] The nanofiber laminate sheet set forth in any one of [1] to [14], wherein the water soluble base layer is composed of a nanofiber portion and a filmy portion, the nanofiber portion and the filmy portion being mingled with each other.
[16] The nanofiber laminate sheet set forth in any one of [1] to [14], wherein the water soluble base layer is a layer of nanofibers in which the nanofibers cohere to each other to form the filmy portions at the intersections.
[17] The nanofiber laminate sheet set forth in [15] or [16], wherein the area ratio of nanofiber portions to filmy portions in a plan view is 90:10 to 10:90.
[18] The nanofiber laminate sheet set forth in [17], wherein the area ratio of nanofiber portions to filmy portions in a plan view is 85:15 to 10:90.
[19] The nanofiber laminate sheet set forth in any one of [1] to [18], having its composition gradually varying from the composition of the water soluble base layer to that of the nanofiber layer in its thickness direction.
[20] The nanofiber laminate sheet set forth I any one of [1] to [18], having a clear boundary between the composition of the water soluble base layer and that of the nanofiber in its thickness direction.
[21] The nanofiber laminate sheet set forth in any one of [1] to [20], containing a volatile functional agent having a vapor pressure of 13.3 Pa or less at 20°C.
[22] A method for making a nanofiber laminate sheet having a nanofiber layer containing a cosmetic component or a medicinal component, and a water soluble base layer located on at least one side of the nanofiber layer, the water soluble base layer being less water-soluble than the nanofiber layer, and the water soluble base layer containing a nanofiber,
   the method comprising electrospinning a solution of a water soluble polymer in a solvent to deposit the water soluble base layer containing a nanofiber on a substrate for deposition in a manner such that part of the nanofiber traveling in air is deposited on the substrate while containing the solvent by decreasing the distance between the tip of a capillary and the substrate and/or increasing the rate of ejecting the water soluble polymer solution thereby to form a filmy portion as well as a nanofiber portion in the water soluble base layer.
[23] A method for making a nanofiber laminate sheet having a nanofiber layer containing a cosmetic component or a medicinal component, and a water soluble base layer located on at least one side of the nanofiber layer, the water soluble base layer being less water-soluble than the nanofiber layer, and the water soluble base layer containing a nanofiber,
   the method comprising electrospinning a water soluble polymer solution to deposit the water soluble base layer containing a nanofiber on a substrate for deposition in a manner such that part of droplets of the solution extruded from a capillary deposit on the substrate before they are drawn into nanofibers thereby to form a filmy portion as well as a nanofiber portion in the water soluble base layer.
[24] A method for making a nanofiber laminate sheet having a nanofiber layer containing a cosmetic component or a medicinal component, and a water soluble base layer located on at least one side of the nanofiber layer, the water soluble base layer being less water-soluble than the nanofiber layer, and the water soluble base layer containing a nanofiber,
   the method comprising simultaneously electrospinning a solution for forming the water soluble base layer from one of two syringes at a gradually increasing rate of ejection starting from 0 up to a predetermined rate and a solution for forming the nanofiber layer from the other syringe at a gradually decreasing rate of ejection starting from a predetermined rate to zero to form the nanofiber laminate sheet which has a composition gradually varying from the composition of the water soluble base layer to the composition of the nanofiber layer.
[25] A method for making a nanofiber laminate sheet having a nanofiber layer containing a cosmetic component or a medicinal component, and a water soluble base layer located on at least one side of the nanofiber layer, the water soluble base layer being less water-soluble than the nanofiber layer and containing an easily volatile functional agent having a vapor pressure exceeding 13.3 Pa at 20°C,
   the method comprising a step of applying the easily volatile functional agent to the nanofiber layer.
[26] A method for making a nanofiber laminate sheet having a nanofiber layer containing a cosmetic component or a medicinal component, and a water soluble base layer located on at least one side of the nanofiber layer, the water soluble base layer being less water-soluble than the nanofiber layer and containing an easily volatile functional agent having a vapor pressure exceeding 13.3 Pa at 20°C,
   the method comprising a step of leaving the easily volatile functional agent to stand close to the nanofiber layer to cause the easily volatile functional agent to transfer to the nanofiber layer.

### Advantageous Effects of Invention

The invention provides a nanofiber sheet, which has hitherto been less than easy to handle, having markedly improved handling so as to be attached to the surface of an object with ease.

### Brief Description of Drawings

[Fig. 1] Fig. 1 is a perspective illustrating part of a device for measuring the dissolving time each of a nanofiber layer and a water soluble base layer.
[Fig. 2] Fig. 2(a), Fig. 2(b), and Fig. 2(c) schematically show the procedure for measuring the dissolving time each of a nanofiber layer and a water soluble base layer by the use of the device shown in Fig. 1.
[Fig. 3] Fig. 3 schematically illustrates an apparatus suitably used to produce the nanofiber laminate sheet according to the invention.
[Fig. 4] Fig. 4 is a scanning electron micrograph of the water soluble base layer of the nanofiber laminate sheet obtained in Example 1.
[Fig. 5] Fig. 5 is a scanning electron micrograph of the water soluble base layer of the nanofiber laminate sheet obtained in Example 2.

### Description of Embodiments

The invention will be described based on its preferred embodiments. The nanofiber laminate sheet of the invention (hereinafter also simply referred to as the laminate sheet) is basically composed of a layer of a nanofiber (hereinafter referred to as a nanofiber layer) and a water soluble base layer. The water soluble base layer is disposed on at least one side of the nanofiber layer. Depending on the use of the laminate sheet, the water soluble base layer may be located on both sides of the nanofiber layer. In this case, the water soluble base layer on one side of the nanofiber layer and that on the other side may be the same or different. Whether provided on one side or both sides, the water soluble base layer preferably adjoins the nanofiber layer. For some uses, between the nanofiber layer and the water soluble base layer there may be a layer different from either.

The nanofiber layer is preferably made solely of a nanofiber. The nanofiber layer may contain other components in addition to a nanofiber. The nanofiber has a thickness usually of 10 to 3000 nm, preferably 10 to 1000 nm, in terms of circle equivalent diameter. The thickness of nanofibers is measured by, for example, observation using a scanning electron microscope (SEM). Specifically, a two-dimensional micrograph at a magnification of 10000 is processed by deleting defects (lumps of nanofibers, intersections of nanofibers, and polymer droplets), randomly choosing ten fibers, drawing a line perpendicular to the longitudinal direction of each fiber chosen, and directly reading the length of the line segment crossing the fiber.

The length of the nanofiber is not critical in the invention and may have any appropriate length depending on the method of nanofiber manufacturing. A nanofiber is called a "fiber" when it is 100 or more times as long as it is thick. The nanofiber may be unidirectionally or randomly aligned in the nanofiber layer. While the nanofiber is generally a solid fiber, a hollow nanofiber or a flattened or ribbon-like hollow nanofiber having a collapsed cross-section is useful as well.

The nanofiber layer may have a thickness decided as appropriate to the intended use of the laminate sheet. For example, for use as attached to human skin, tooth, or gum, the thickness of the nanofiber layer is preferably 50 nm to 1 mm, more preferably 500 nm to 500 µm. The thickness of the nanofiber layer may be measured using a contact thickness gauge Litematic VL-50A from Mitutoyo Corp. with a spherical carbide contact point of 5 mm in radius. A load of 0.01 N is applied to the sheet in the thickness measurement.

The nanofiber layer may have a basis weight decided as appropriate to the intended use of the laminate sheet. For example, for use as attached to human skin, tooth, or gum, the basis weight of the nanofiber layer is preferably 0.01 to 100 g/m², more preferably 0.1 to 50 g/m².

The nanofibers in the nanofiber layer are bonded to one another at the intersections thereof or intertwined with one another, whereby the nanofiber layer is self-supporting to retain its sheet form. Whether the nanofibers are bonded to or intertwined with one another depends on the method of manufacturing the nanofiber layer.

The nanofiber is prepared from a polymer. The polymer may be a naturally occurring polymer or a synthetic polymer. The polymer is preferably water soluble.

As used herein, the term "water soluble polymer" denotes a polymer having solubility such that, when a sample polymer weighing 1 g is immersed in 10 g of ion-exchanged water for 24 hours at 23°C and atmospheric pressure, at least 0.5 g of the immersed polymer dissolves in the ion-exchanged water.

Examples of the water soluble polymer include naturally occurring polymers, such as mucopolysaccharides, e.g., pullulan, hyaluronic acid, chondroitin sulfate, poly-γ-glutamic acid, modified corn starch, β-glucan, gluco-oligosaccharides, heparin, and keratosulfate, cellulose, pectin, xylan, lignin, glucomannan, galacturonic acid, psyllium seed gum, tamarind seed gum, gum arabic, tragacanth gum, soybean water-soluble polysaccharides, alginic acid, carrageenan, laminaran, agar (agarose), fucoidan, methyl cellulose, hydroxypropyl cellulose, and hydroxypropylmethyl cellulose; and synthetic polymers, such as partially saponified polyvinyl alcohol (usable in the absence of a crosslinking agent hereinafter described), low-saponified polyvinyl alcohol, polyvinylpyrrolidone (PVP), polyethylene oxide, and sodium polyacrylate. These water soluble polymers may be used either individually or in combination of two or more thereof. Preferred of them are pullulan and synthetic polymers, such as partially saponified polyvinyl alcohol, low-saponified polyvinyl alcohol, polyvinylpyrrolidone, and polyethylene oxide, in view of ease of conversion to nanofibers.

The nanofiber contains a cosmetic component or a medicinal component. As attached to, e.g., human skin, tooth, or gum, the nanofiber layer containing such an active ingredient produces the advantageous effect attributed to the active ingredient.

Examples of the cosmetic or medicinal component include a blood circulation improving agent selected from acid mucopolysaccharides, chamomile, buckeye, ginkgo, witch hazel extract, vitamin E, nicotinic acid derivatives, and alkaloid compounds; an edema reducing agent selected from buckeye, flavone derivatives, naphthalenesulfonic acid derivatives, anthocyanin, vitamin P, calendula, concholytic acid, silanol, terminalia, visnaga, and majus; a slimming agent selected from aminophylline, tea extract, caffeine, xanthine derivatives, inositol, dextransulfuric acid derivatives, buckeye, aescin, anthocyanidin, organoiodine compounds, hypericum, Filipendula multijuga, field horsetail, rosemary, ginseng, ivy, thiomucase, and hyaluronidase; a painkiller selected from indometacin, diclofenac, d1-camphor, flurbiprofen, ketoprofen, capsicum extract, piroxicam, felbinac, methyl salicylate, and glycol salicylate; a moisturizing agent selected from polyols, ceramids, and collagens; a peeling gent including a protease; a depilatory agent including calcium thioglycolate; and an autonomic regulatory agent including γ-oryzanol. Additionally, extracts of Japanese elk horn ceder (Thujopsis dolabrata), balloon flower root, eucalyptus, birch, ginger, yuzu (Citrus junos), etc.; tranexamic acid, allantoin, stearyl glycyrrhetinate, niacinamide, 1-menthol, and vitamins such as vitamin C are also included in the cosmetic or medicinal component. The content of the active ingredient in the nanofiber is usually in the range of from 0.01% to 70% by mass, while varying according to the kind. The active ingredient content may be determined by liquid chromatography or thermogravimetry.

The nanofiber may further contain other components in addition to the above described components. Such components include a crosslinking agent, a pigment, a filler, a surfactant, an antistatic agent, and a foaming agent. A crosslinking agent is used to crosslink and insolubilize, for example, the partially saponified polyvinyl alcohol described above. A pigment is used to color the nanofiber.

The water soluble base layer provided on at least one side of the nanofiber layer is constructed of a layer containing a water soluble material. The term "water soluble" as used herein is as previously defined. One of the characteristics of the water soluble base layer is a difference in water solubility from the nanofiber layer. Specifically, the water solubility of the water soluble base layer is relatively lower than that of the nanofiber layer. In other words, the water soluble base layer is less water-soluble than the nanofiber layer. Therefore, when the laminate sheet of the invention is taken, for example, between fingers and attached to a desired part, dissolution of the nanofiber layer due to the water, e.g., of perspiration is prevented by the water soluble base layer until it is successfully attached. The less water-solubility of the water soluble base layer than that of the nanofiber layer brings about improvement on the handling of the nanofiber layer with respect to the behavior to water. Since the water soluble base layer is water soluble per se, it dissolves in water and disappears after the elapse of a prescribed period of time so that it gives no adverse influences on the nanofiber layer. In the case where the water soluble base layer is provided on both sides of the nanofiber layer, the nanofiber layer is blocked by the two water soluble base layers, so that the active ingredient in the nanofiber layer is slowly released to advantage for a prolonged period of time.

To say that the water soluble base layer is "less water soluble" than the nanofiber layer is equivalent to say that the water soluble base layer is "less soluble" than the nanofiber layer. The solubility difference between the water soluble base layer and the nanofiber layer is evaluated by, for example, methods I or II below.

### Method I:

A sample sheet measuring 4 cm by 6 cm is cut out of each of the water soluble base layer and the nanofiber layer before they are laminated with each other. The sample sheet is stuck to a 0.2 mm thick polyethylene terephthalate film using a double sided adhesive tape. An ultrasonic humidifier KX-80UP from CCP Co., Ltd. having a 30 mm diameter outlet is turned on to generate water vapor at a rate of 90 ml/hr. The sample sheet is fixed at a distance of 10 mm from the outlet, and the time required for the sheet to turn transparent as observed with naked eyes is taken as a dissolving time. The dissolving time of the nanofiber layer as measured by the method I is preferably 120 seconds or shorter, more preferably 10 to 60 seconds. The dissolving time of the water soluble base layer as measured by the method I is preferably 30 seconds or longer, more preferably 35 to 180 seconds, even more preferably 40 to 100 seconds. The dissolving time ratio of water soluble base layer to nanofiber layer is preferably 1.1 or greater, more preferably 1.1 to 50. The method I is also applicable to a nanofiber laminate sheet after laminating the water soluble base layer and the nanofiber layer to each other.

### Method II:

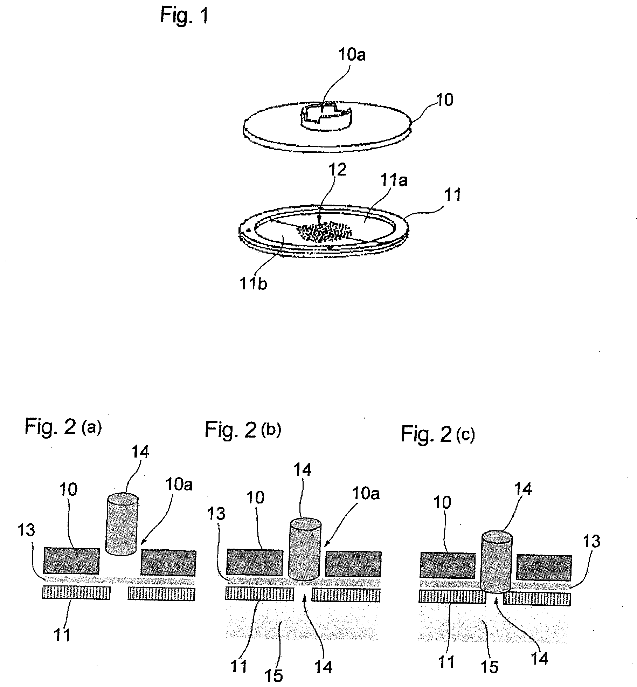

An orally disintegrating tablet tester ODT-101 from Toyama Sangyo Co., Ltd. was used. As shown in Fig. 1, the tester has a plate-shaped, sample fixing frame 10 having a circular hole 10a at the center and a detection sheet 11. The detection sheet 11 is composed of a pair of semicircular metal sheets 11a and 11b with their straight edges facing each other at a prescribed clearance therebetween to form a slit (not shown). The detection sheet 11 has a number of small holes 12 in its central portion. Each of the water soluble base layer and the nanofiber layer before lamination is cut to measure 4 cm by 4 cm to prepare a sample sheet 13. The sample sheet 13 is fixed between the sample fixing frame 10 and the detection sheet 11 as shown in Fig. 2(a). A metal plumb 14 is positioned above the circular hole of the sample fixing frame 10. As shown in Fig. 2(b), the plumb 14 is, while being rotated in a horizontal plane, let down into the circular hole 10a, where the rotating plumb 14 is brought into contact with the exposed part of the sample sheet 13. Simultaneously with the contact, water 15 is made to seep through the slit 14 from the back side of the detection sheet 11 to wet the sample sheet 13. This point of time is taken as a starting point of measurement. The sample sheet 13 gradually dissolves by the action of wetting with water and rotation of the rotating plumb 14. The time point when the sample sheet 13 dissolves completely to allow the lower side of the plumb 14 to touch the detection sheet 11 as shown in Fig. 2(c) is the end of measurement. The time from the start to end of measurement is the dissolving time. The dissolving time of the nanofiber layer as measured by the method II is preferably 100 seconds or less, more preferably 0.1 to 50 seconds. The dissolving time of the water soluble base layer as measured by the method II is preferably 5 seconds or more, more preferably 5 to 600 seconds, even more preferably 5 to 90 seconds. The dissolving time ratio of water soluble base layer to nanofiber layer is preferably 1.1 or greater, more preferably 1.1 to 200.

The following advantages accrue from the dissolving times of the nanofiber layer and the water soluble base layer being in the respective ranges recited. The laminate sheet of the nanofiber layer and the water soluble base layer is easy to handle by hand. When the nanofiber laminate sheet is attached to an object with the nanofiber layer inside, the nanofiber layer dissolves earlier than the water soluble base layer so that the cosmetic or medicinal component in the nanofiber layer may be imparted to the object, and the water soluble base layer covers the cosmetic or medicinal component to prevent the cosmetic or medicinal component from running over other than the object. Improvement is also expected on the penetrability of the cosmetic or medicinal component into the object. As time advances, the water soluble base layer also dissolves, leaving no waste to be disposed of. This will provide a better usage of the laminate sheet.

A water soluble base layer having lower water solubility than a nanofiber layer is obtainable, for example, by means (a) in which the water soluble base layer is made from a material having lower water solubility than the material making the nanofiber except a water insoluble material. When the water soluble base layer is made of the same material as used to make the nanofiber layer, means (b) is adopted, in which a water soluble base layer is formed to have a smaller specific surface area than the nanofiber layer. The means (a) and (b) may be used in combination. The water soluble base layer may have an air permeable structure.

In using the means (b), the water soluble base layer is formed of a poreless or porous film or a mesh. The water solubility of the water soluble base layer is adjusted as desired by properly selecting the thickness and basis weight of the film or the size and number of the pores, and the like. The thickness, the basis weight, and the size and number of the pores are decided by those skilled in the art within the range of ordinary knowledge. Specific examples of useful water soluble base layers are films of polysaccharide materials, such as oblate and pullulan, polyvinyl alcohol, agar, or carrageenan. The film does not always need to be totally water soluble. It is only necessary that the film disintegrate easily in the presence of water. That is, when the water soluble base layer is water soluble, the layer may dissolve with water completely or the layer may lose its sheet form on contact with water.

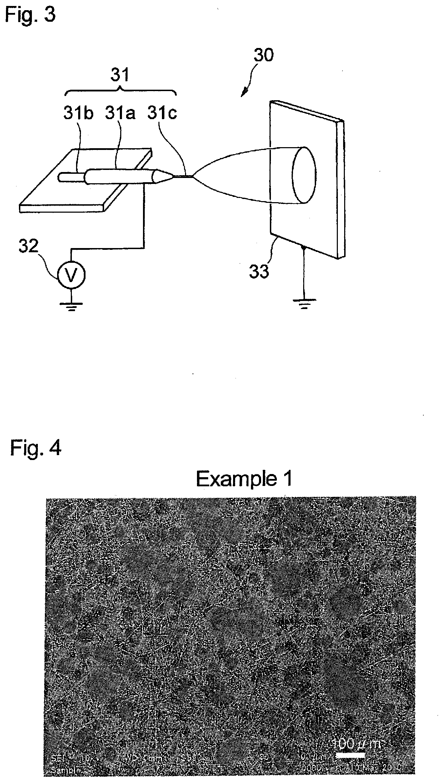

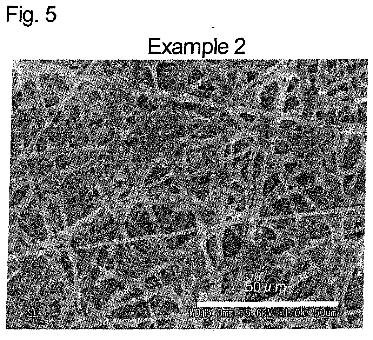

When the means (b) is adopted and when the water soluble base layer contains a nanofiber, the water soluble base layer may be composed of nanofiber portions mingled with portions causing reduction of specific surface area of the water soluble base layer, for example, the cohesive filmy portions shown in Figs. 4 and 5 hereinafter described. When the water soluble base layer is constructed of nanofiber portions and filmy portions, the area ratio of the nanofiber portions to the filmy portions in a plan view is preferably 90:10 to 10:90, more preferably 85:15 to 10:90, in terms of desired water solubility. The area ratio is determined by observing a water soluble base layer under an SEM at a magnification of 100 and processing the micrograph by background extraction and automatic binarization using image processing software Image-ProPlus from Roper Industries, Japan. In the case when no filmy portions appear in spots, the water soluble base layer is then observed under an SEM at a magnification increased to 1000, and the micrograph is processed in the same manner to obtain the area ratio.

The active ingredient contained in the nanofiber is not precluded from being present in the water soluble base layer. However, no particular effect is expected of the presence of the active ingredient in the water soluble base layer. Therefore, the water soluble base layer does not usually have to contain the active ingredient.

The laminate sheet of the invention may contain a volatile functional agent. A volatile functional agent may be present in either one or both of the nanofiber layer and the water soluble base layer of the laminate sheet. The volatile functional agent is at least one member selected from the group consisting of a fragrance, a whitening agent, and a taste corrector. As used herein, the "fragrance" means a substance capable of imparting a pleasant scent to air at ambient temperature and atmospheric pressure and having a "fragrant function". As used herein, the "whitening agent" means a substance which is, when applied to human skin, capable of whitening the skin or maintaining the skin in a youthful and healthy condition and has a "whitening function". As sued herein, the "taste corrector" means a substance capable of changing or diminishing a taste and having a "taste correcting function". For example, a taste corrector may change bitterness or sourness to another taste, e.g., sweetness, or reducing a taste. These functional agents are volatile substances vaporizing at ambient temperature and atmospheric pressure. As used herein, the term "ambient temperature and atmospheric pressure" usually means a condition of a temperature of 23°C and an atmospheric pressure of 101.325 kPa.

The volatile functional agent preferably has a vapor pressure at 20°C of 13.3 Pa or less, more preferably 0.0013 to 10.7 Pa, even more preferably 0.0133 to 6.7 Pa. With the vapor pressure at 20°C of the volatile functional agent falling within that range, the laminate sheet of the invention will exhibit a useful function attributed to the volatile functional agent at ambient temperature and atmospheric pressure. For instance, a laminate sheet of the invention containing a fragrance as a volatile functional agent is a fragranced sheet capable of releasing a fragrance into air at ambient temperature and atmospheric pressure thereby to make a user feel exhilarated, refreshed, cleaned, or relaxed and also produce a deodorizing effect, an anesthetic (analgesic) effect, or a like effect.

When the laminate sheet of the invention is produced by electrospinning (a method for producing the nanofiber, hereinafter described) using a stock polymer solution containing a volatile functional agent whose vapor pressure at 20°C exceeds 13.3 Pa (hereinafter also referred to as an easily volatile functional agent), the easily volatile functional agent tends to vaporize during electrospinning because of its high volatility. It can follow that the resulting nanofiber fails to retain a sufficient amount of the easily volatile functional agent for performing the function expected of the use of the functional agent, for example, a fragrant function of an easily volatile fragrance. Nevertheless, it is possible to impart the function of such an easily volatile functional agent to a nanofiber by timely adding the easily volatile functional agent (the timely addition will be described later). The vapor pressure of a volatile functional agent is obtained from the data base provided by Research Institute for Fragrance Materials.

Examples of fragrances that can be used as a volatile functional agent include vanillin, methyl jasmonate, γ-undecalactone, and phenylethyl alcohol. These volatile functional agents may be used either individually or in combination of two or more thereof.

The volatile functional agent content in the laminate sheet is preferably 0.001% to 30%, more preferably 0.01% to 5%, by mass. The recited range of the volatile functional agent content assures production of a nanofiber performing a useful function of the volatile functional agent, such as fragrance release, and achieves reduction in the amount of the volatile functional agent to be used, leading to a reduced cost of production.

A preferred method for producing the laminate sheet of the invention will then be described. The nanofiber layer of the laminate sheet is suitably produced by, for example, depositing a nanofiber by electrospinning on a smooth surface of a substrate. Fig. 3 illustrates an apparatus 30 for carrying out electrospinning deposition. The apparatus 30 includes a syringe 31, a high voltage supply 32, and a conductive collector 33. The syringe 31 has a cylinder 31a, a plunger 31b, and a capillary 31c. The capillary 31c has an inner diameter of about 10 to 1000 µm. The cylinder 31a is filled with a stock solution containing a polymer and an active ingredient, the raw material of a nanofiber. The solvent of the stock solution is selected from water, an organic solvent, and a mixture of water and a water-compatible organic solvent according to the kind of the polymer. The high voltage supply 32 is, for example, a 10 to 30 kV direct voltage source. The positive pole of the high voltage supply 32 is electrically connected to the polymer solution in the syringe 31, with the negative pole grounded. The conductive collector 33 is, e.g., a metal plate that is grounded. The distance between the tip of the capillary 31c of the syringe 31 and the conductive collector 33 is set at, e.g., about 30 to 300 mm. The apparatus 30 shown in Fig. 3 may be operated in the atmosphere. The operative environment is not particularly limited and may be, for example, 20° to 40°C and 10 to 50% RH.

With a voltage applied between the syringe 31 and the conductive collector 33, the plunger 31b of the syringe 31 is slowly forced into the cylinder 31a to eject the polymer solution from the tip of the capillary 31c. The solvent of the extruded solution vaporizes, and the solute (i.e., the polymer) is drawn into a nanofiber while solidifying and attracted onto the conductive collector 33 by the difference in electrical potential. From the principle of the production process, the thus formed nanofiber is a continuous filament of infinite length. A hollow nanofiber is obtained by, for example, using a double barreled capillary as the capillary 31c and feeding incompatible solutions in the core and the sheath.

When the water soluble base layer is a poreless or porous film or a mesh, such a poreless or porous film or a mesh is used as the substrate on which the nanofiber layer is deposited to make a laminate sheet having one water soluble base layer and one nanofiber layer.

When the water soluble base layer contains a nanofiber, a water soluble base layer containing a nanofiber is formed on the surface of a separately prepared substrate, and a nanofiber layer is then formed thereon to provide a laminate sheet having one water soluble base layer and one nanofiber layer. If desired, a water soluble base layer may be formed on the thus formed nanofiber layer to provide a three-layered laminate sheet having the water soluble base layer on both sides of one nanofiber layer. Formation of the water soluble base layer and the nanofiber layer may be achieved by, for example, using two syringes. A solution for forming the water soluble base layer is ejected from one of the syringes, and thereafter a solution for forming the nanofiber layer is ejected from the other syringe. In this case, the resulting laminate sheet exhibits a clear boundary between the composition of the water soluble base layer and that of the nanofiber layer in the laminate sheet thickness direction.

Instead of the above discussed system of ejection, the two syringes may operate simultaneously. A solution for forming the water soluble base layer is ejected from one of the syringes at a gradually increasing rate of ejection starting from 0 up to a predetermined rate. A solution for forming the nanofiber layer is ejected from the other syringe at a gradually decreasing rate of ejection starting from a predetermined rate to zero. A laminate sheet obtained by this system has a composition gradually varying from the composition of the water soluble base layer to that of the nanofiber layer in its thickness direction. Alternately, a solution for forming the nanofiber layer is ejected from one of the syringes at a gradually increasing rate of ejection starting from 0 up to a predetermined rate and, at the same time, a solution for forming the water soluble base layer is ejected from the other syringe at a gradually decreasing rate of ejection starting from a predetermined rate to zero. When the laminate sheet does not have a distinct boundary between the water soluble base layer and the nanofiber layer as with the cases discussed above, the nanofiber layer has a prolonged dissolving time, which is advantageous in that the active ingredient is slowly released over an extended period of time.

As stated earlier, when the water soluble base layer contains a nanofiber, it is preferred that the water soluble base layer be composed of nanofiber portions and portions causing reduction of specific surface area of the water soluble base layer, for example, cohesive filmy portions mingled with each other. Formation of the filmy portions as well as the nanofiber portions by the above discussed electrospinning process is achieved by, for example, the following methods (i) and (ii).

### Method (i):

In carrying out electrospinning, the distance between the tip of the capillary 31C and the substrate for deposition is reduced and/or the rate of ejection is increased so that part of the nanofibers traveling in air may deposit on the substrate for deposition while containing the solvent.

### Method (ii):

In carrying out electrospinning, the distance between the tip of the capillary 31c and the substrate for deposition is reduced and/or the rate of ejection is increased so that part of droplets of the solution extruded from the capillary 31c deposit on the substrate for deposition before they are drawn into a nanofiber.

In following the method (i), since part of the nanofibers deposit while containing the solvent, the nanofibers dissolve at their intersections to become filmy to reflect the dissolved state in the resulting water soluble base layer.

When the method (ii) is followed, since part of the solution droplets deposit without being drawn, the resulting water soluble base layer includes not only the nanofibers but also dot-shaped filmy portions to reflect the droplets. The dot-shaped filmy portions exist independently of the intersections of the nanofibers. The size of the dot-shaped filmy portion is 5 to 500 µm in terms of circle-equivalent diameter, while varying depending on the electrospinning conditions.

Thus, a water soluble base layer having desired water solubility is obtained by properly adjusting the distance between the tip of the capillary 31C and the substrate for deposition and/or the rate of ejection of the solution according to the method (i) or (ii).

In the case when the laminate sheet of the invention contains the above described volatile functional agent, the volatile functional agent is incorporated into the polymer solution used to form a nanofiber layer by electrospinning. The same applies to the case when the water soluble base layer is made of nanofibers. When a nanofiber is formed by electrospinning using the polymer solution containing the volatile functional agent, vaporization of the volatile functional agent is effectively prevented because the vapor pressure at 20°C of the volatile functional agent is 13.3 Pa or less as previously noted. On the other hand, when a volatile functional agent whose vapor pressure at 20°C exceeds 13.3 Pa (hereinafter also referred to as an easily volatile functional agent) is used, the easily volatile functional agent is allowed to be imparted to a nanofiber by timely adding the easily volatile functional agent. More specifically, a laminate sheet provided with the function of an easily volatile functional agent is obtainable according to the following methods A and B.

Method A: A method for producing a laminate sheet including a step of making a nanofiber layer containing a polymer nanofiber (nanofiber layer making step) and a step of applying a solution containing an easily volatile functional agent to the nanofiber layer (solution addition step).

Method B: A method for producing a laminate sheet including a step of making a nanofiber layer containing a polymer nanofiber (nanofiber layer making step) and a step of leaving an easily volatile functional agent to stand close to the nanofiber layer for a prescribed period of time (easily volatile functional agent transfer step).

The nanofiber layer making step of the methods A and B is carried out by the electrospinning process shown in Fig. 3. The easily volatile functional agent may be applied to the nanofiber layer either before or after the water soluble base layer is formed on the nanofiber layer.

The solution containing an easily volatile functional agent for use in the solution addition step of the method A is prepared by dissolving or dispersing the easily volatile component in a solvent. The solvent is preferably uninfluential on the nanofiber, specifically the water soluble polymer making up the nanofiber. For example, the solvent is selected from water, an organic solvent, and a mixture of water and a water-compatible organic solvent according to the kind of the polymer. Application of the solution containing the easily volatile functional agent to the nanofiber layer may be achieved by, for example, spraying the solution to the nanofiber layer or immersing the nanofiber layer in the solution.

The easily volatile functional agent transfer step of the method B is a step in which the nanofiber layer and the easily volatile functional agent are brought close to but not in contact with each other thereby to cause the vapor of the easily volatile functional agent to transfer to the nanofiber layer. If the nanofiber layer and the easily volatile functional agent are brought into contact with each other, the water soluble polymer constituting the nanofiber can dissolve or swell to lose its fibrous form. The easily volatile functional agent placed close to the nanofiber layer may be exposed to open air or be enclosed in an air permeable bag or like enclosure. Unintentional direct contact of the nanofiber layer with the easily volatile functional agent is certainly avoided by enclosing the easily volatile functional agent. The period of time that the easily volatile functional agent is left to stand close to the nanofiber layer is decided as appropriate to the type of the easily volatile functional agent and the like. In general, the higher the volatility of the functional agent, the shorter the time needed.

The thus obtained laminate sheet of the invention is used as attached to, for example, the skin, tooth, or gum of humans, the skin, tooth, or gum of non-human mammals, and the surface of plant parts, such as foliage. The laminate sheet which is composed of one nanofiber layer and one water soluble base layer is attached to an object with the nanofiber layer inside. The laminate sheet which has a water soluble base layer on either side of the nanofiber layer is attached to an object with either water soluble base layer inside.

Before the laminate sheet is applied to the surface of an object, the surface of the object may be wetted with a liquid so that the laminate sheet successfully adheres to the surface with the surface tension taken advantage of. Alternately, the surface of the laminate sheet (the side to face the object) may be wetted with a liquid.

The surface of an object may be wetted by, for example, spreading or spraying a liquid of various kinds to the surface. The liquid to be spread or sprayed is a substance containing a component that is liquid at a temperature of attaching the laminate sheet and having a viscosity of about 5000 mPa·s or less at that temperature as measured with a corn-plate rotational viscometer. Such a liquid is exemplified by water, an aqueous liquid, e.g., an aqueous solution or an aqueous dispersion, a nonaqueous solvent, and a solution or dispersion containing a nonaqueous solvent. Emulsions, including O/W emulsions and W/O emulsions, and liquids thickened with a thickener, such as a thickening polysaccharide, are also useful. More specifically, in the case when the laminate sheet of the invention is a cosmetic article to be attached to human skin, a skin lotion or a beauty cream is useful as a liquid wetting the surface of the object (skin).

While the invention has been described with reference to its preferred embodiments, it should be understood that the invention is not limited to these embodiments. For example, while the method for producing the nanofiber has been described with particular reference to electrospinning deposition, the method for producing the nanofiber is not limited thereto.

While, according to the electrospinning technique shown in Fig. 3, the nanofiber formed is deposited on the conductive collector 33 of plate shape, a conductive rotating drum may be used instead of the plate-shaped collector, in which case the nanofiber is deposited on the peripheral surface of the rotating drum.

### Examples

The invention will now be illustrated in greater detail by way of Examples, but it should be understood that the scope of the invention is not limited thereto. Unless otherwise noted, all the percents are by mass.

### Example 1

Pullulan available from Hayashibara Shoji Inc. was used as a nanofiber layer material. Pullulan was dissolved in 80°C water to make a 15% solution. The solution was cooled to room temperature, and ascorbic acid was dissolved therein in a concentration of 5% to prepare a stock solution for electrospinning. The stock solution was electrospun by the use of the apparatus shown in Fig. 3 in which 30 µm thick aluminum foil was placed on the surface of the collector 33 to form a 53 µm-thick water-soluble nanofiber layer on the aluminum foil. The electrospinning conditions are described below.
Applied voltage: 25 kV
Capillary-collector distance: 185 mm
Rate of ejection: 1.0 ml/hr
Environment: 26°C, 40% RH

Pullulan available from Hayashibara Shoji Inc. was used as a water soluble base layer material. Pullulan was dissolved in 80°C water to make a 15% solution, which was cooled to room temperature to prepare a stock solution. The stock solution was electrospun onto the surface of the water-soluble nanofiber layer prepared above to form a 48 µm-thick water-soluble base layer thereby to produce a laminate sheet. The resulting laminate sheet had a thickness of 101 µm. The electrospinning conditions are described below. The rate of ejecting the stock solution was increased over that used in the formation of the nanofiber layer so that part of the solution droplets deposited without being drawn to create dot-shaped filmy portions reflecting the droplets.
Applied voltage: 25 kV
Capillary-collector distance: 185 mm
Rate of ejection: 15.0 ml/hr
Environment: 26°C, 40% RH

There was thus obtained a laminate sheet having one nanofiber layer and one water soluble base layer composed of nanofibers. The nanofibers in each layer were found to have the thickness shown in Table 1 below as a result of SEM observation. An SEM image of the water soluble base layer of the laminate sheet is shown in Fig. 4. As is apparent from Fig. 4, many dot-shaped filmy portions are formed in the water soluble base layer. The ratio of the nanofiber portions in the water soluble base layer was determined by the method described supra. The results obtained are also shown in Table 1.

### Example 2

A nanofiber layer was formed using the same stock solution and the same substrate under the same conditions as in Example 1. The thickness of the nanofiber layer was 53 µm. A water soluble base layer was formed by electrospinning the same stock solution as used in Example 1 onto the nanofiber layer to a thickness of 51 µm. The electrospinning conditions are described below. Compared with the conditions employed in the nanofiber layer formation, the distance between the tip of the capillary 31c and the nanofiber layer was reduced, and the rate of ejecting the stock solution was increased, so that part of the nanofibers deposited while containing the solvent to create filmy portions at the intersections of the nanofibers where the nanofibers dissolved. The thus obtained nanofiber laminate sheet had a thickness of 104 µm.
Applied voltage: 25 kV
Capillary-collector distance: 90 mm
Rate of ejection: 4.0 ml/hr
Environment: 26°C, 40% RH

There was thus obtained a laminate sheet having one nanofiber layer and one water soluble base layer composed of nanofibers. The nanofibers in each layer were found to have the thickness shown in Table 1 below as a result of SEM observation. An SEM image of the water soluble base layer of the laminate sheet is shown in Fig. 5. As is apparent from Fig. 5, many filmy portions reflecting the dissolved state of nanofibers are formed at the intersections of the nanofibers in the water soluble base layer. The ratio of the nanofiber portions in the water soluble base layer was determined by the method described supra. The results obtained are also shown in Table 1.

### Example 3

A commercially available film of oblate (basis weight: 25 g/m²; thickness: 30 µm) was used as a water soluble base layer. A nanofiber layer was formed by electrospinning using the same stock solution and the same conditions as in Example 1 onto the water soluble base layer as a substrate to a thickness of 53 µm. The thickness of the resulting nanofiber laminate sheet was 83 µm. There was thus obtained a laminate sheet composed of one nanofiber layer and one water soluble base layer of film form. The thickness of the nanofibers in the nanofiber layer was measured by SEM observation. The results obtained are shown in Table 1.

### Example 4

A nanofiber layer was formed using the same stock solution and the same substrate under the same conditions as in Example 1. The thickness of the nanofiber layer was 53 µm. A water soluble base layer was formed by electrospinning the same stock solution as used in Example 1 onto the nanofiber layer to a thickness of 55 µm. The electrospinning conditions are described below. Compared with the conditions employed in the nanofiber layer formation, the rate of ejecting the stock solution was increased so that part of the droplets deposited without being drawn to create dot-shaped filmy portions reflecting the droplets. The thus obtained nanofiber laminate sheet had a thickness of 108 µm.
Applied voltage: 25 kV
Capillary-collector distance: 185 mm
Rate of ejection: 11.0 ml/hr
Environment: 26°C, 40% RH

There was thus obtained a laminate sheet having one nanofiber layer and one water soluble base layer composed of nanofibers. The nanofibers in each layer were found to have the thickness shown in Table 1 below as a result of SEM observation. The ratio of the nanofiber portions in the water soluble base layer was determined by the method described supra. The results obtained are also shown in Table 1.

### Example 5

A polyvinyl alcohol film Hi-Selon M-250 from The Nippon Synthetic Chemical Industry (thickness: 22 µm) was used as a water soluble base layer. A nanofiber layer was formed by electrospinning using the same stock solution and the same conditions as used in Example 1 on the water soluble base layer as a substrate to a thickness of 53 µm. The thickness of the resulting nanofiber laminate sheet was 75 µm. There was thus obtained a laminate sheet composed of one nanofiber layer and one water soluble base layer of film form. The thickness of the nanofibers in the nanofiber layer was measured by SEM observation. The results obtained are shown in Table 1.

### Comparative Example 1

A nanofiber sheet having a thickness of 110 µm was formed by electrospinning using the same stock solution, conditions, and the substrate as in Example 1.

### Evaluation:

The sheets obtained in Examples and Comparative Example were each evaluated for solubility of the nanofiber layer and the water soluble base layer and handling of the sheet in accordance with the methods described below. The results of evaluation are shown in Table 1.

### (1) Solubility of nanofiber layer and water soluble base layer

### Method I:

A nanofiber layer and a water soluble base layer were formed separately. A sample sheet measuring 4 cm by 6 cm was cut out of each of the water soluble base layer and the nanofiber layer. The sample sheet was stuck to a 0.2 mm thick polyethylene terephthalate film using a double sided adhesive tape. An ultrasonic humidifier KX-80UP from CCP Co., Ltd. having a 30 mm diameter outlet was turned on to generate water vapor at a rate of 90 ml/hr. The sample sheet was fixed at a distance of 10 mm from the outlet, and the time required for the sheet to turn transparent as observed with naked eyes was taken as a dissolving time I. The measurement was made in triplicate to obtain an average time.

### Method II:

An orally disintegrating tablet tester ODT-101 from Toyama Sangyo Co., Ltd. shown in Figs. 1 and 2 was used. Each of the water soluble base layer and the nanofiber layer which were prepared separately was cut to measure 4 cm by 4 cm to prepare a sample sheet. The sample sheet was fixed to the tester. The plumb 14 had a diameter of 20 mm and a weight of 15 g. The rotational speed of the plumb 14 was 5 rpm. The water temperature was 23°C. The dissolving time as measured by this method was taken as a dissolving time II.

### (2) Handling of Sheet

The surface of the water soluble base layer (the nanofiber layer in Comparative Example 1) was pressed for 3 seconds with a finger having been thoroughly wiped with a cotton wipe to remove any moisture. After the finger was removed, the change of the surface of the sheet was observed with naked eyes and rated as follows.
Good: A slight change is observed, such as formation of a small hole in part of the region where the finger has been placed.
Poor: A change is observed, such as formation of small holes over the entire region where the finger has been placed.

**Table 1**

| | | Example 1 | Example 2 | Example 3 | Example 4 | Example 5 | Comparative Example 1 |
|---|---|---|---|---|---|---|---|
| Nanofiber Layer | Kind | pullulan + ascorbic acid | pullulan + ascorbic acid | pullulan + ascorbic acid | pullulan + ascorbic acid | pullulan + ascorbic acid | pullulan + ascorbic acid |
| | Active Ingredient Concentration (%) | 26 | 26 | 26 | 26 | 26 | 26 |
| | Fiber Thickness (nm) | 452 | 452 | 452 | 452 | 452 | 452 |
| | Layer Thickness (µm) | 53 | 53 | 53 | 53 | 53 | 110 |
| | Dissolving Time I (s) | 25 | 25 | 25 | 25 | 25 | 32 |
| | Dissolving Time II (s) | 6.22 | 6.22 | 6.22 | 6.22 | 6.22 | 7.98 |
| Water Soluble Base Layer | Kind | nanofiber layer (pullulan) + dot-shaped filmy portions | nanofiber layer (pullulan) + filmy portions by dissolving at intersections | oblate film | nanofiber layer (pullulan) + dot-shaped filmy portions | polyvinyl alcohol film | - |
| | Fiber Thickness (nm) | 674 | 1366 | - | 587 | - | |
| | Layer Thickness (µm) | 48 | 51 | 30 | 55 | 22 | |
| | Ratio of Nanofiber Portions | 46 | 75 | - | 82 | - | |
| | Dissolving Time I (s) | 81 | 45 | ≥180 | 58 | ≥180 | |
| | Dissolving Time II (s) | 9.6 | 8.1 | ≥600 | 8.4 | 95.6 | |
| Evaluation | Handling | good | good | good | good | good | poor |

As is apparent from the results in Table 1, the laminate sheets of Examples (of the invention) prove to have markedly improved handling properties with respect to dissolving with water as compared with the nanofiber sheet of Comparative Example.

## Claims

1. A nanofiber laminate sheet comprising a nanofiber layer containing a cosmetic component or a medicinal component, and a water soluble base layer located on at least one side of the nanofiber layer, the water soluble base layer being less water-soluble than the nanofiber layer, wherein the term "water soluble" denotes a solubility such that, when a sample weighing 1g is immersed in 10g of ion-exchanged water for 24 hours at 23°C and atmospheric pressure, at least 0.5g of the immersed sample dissolves in the ion-exchanged water.

2. The nanofiber laminate sheet according to claim 1, wherein the nanofiber is prepared from a polymer, which is water soluble.

3. The nanofiber laminate sheet according to claim 1 or 2, wherein the water soluble base layer and the nanofiber comprise the same material,
the water soluble base layer has a smaller specific surface area than the nanofiber layer, and
the water soluble base layer comprises a poreless film, a porous film, or a mesh.

4. The nanofiber laminate sheet according to any one of claims 1 to 3, wherein the water soluble base layer is a film prepared from a polysaccharide material, polyvinyl alcohol, agar, or carrageenan.

5. The nanofiber laminate sheet according to any one of claims 1 to 4, the content of the cosmetic component or the medicinal component in the nanofiber is in the range of from 0.01% to 70% by mass.

6. The nanofiber laminate sheet according to any one of claims 1 to 5, wherein the water soluble base layer is constructed of a nanofiber portion and a filmy portion, the nanofiber portion and the filmy portion being mingled with each other.

7. The nanofiber laminate sheet according to any one of claims 1 to 6, wherein the water soluble base layer is a layer of nanofibers in which nanofibers cohere to each other to form the filmy portions at their intersections.

8. The nanofiber laminate sheet according to claim 6 or 7, wherein the area ratio of nanofiber portions to filmy portions in a plan view is 90:10 to 10:90.

9. The nanofiber laminate sheet according to any one of claims 1 to 8, having its composition gradually varying from the composition of the water soluble base layer to that of the nanofiber layer in its thickness direction.

10. The nanofiber laminate sheet according to any one of claims 1 to 8, having a clear boundary between the composition of the water soluble base layer and that of the nanofiber layer in its thickness direction.

11. The nanofiber laminate sheet according to any one of claims 1 to 10, containing a volatile functional agent having a vapor pressure of 13.3 Pa or less at 20°C.

12. The nanofiber laminate sheet according to claim 11, wherein the volatile functional agent is at least one member selected from the group consisting of a fragrance, a whitening agent, and a taste corrector.

13. The nanofiber laminate sheet according to claim 11 or 12, wherein the content of the volatile functional agent is 0.001% to 30% by mass.

14. The nanofiber laminate sheet according to any one of claims 1 to 13, which is for use as a sheet that is to be attached to human skin, tooth, or gum, and
wherein the thickness of the nanofiber layer is 50 nm to 1 mm.

15. The nanofiber laminate sheet according to any one of claims 1 to 14, which is for use as a sheet that is to be attached to human skin, tooth, or gum, and
wherein the basis weight of the nanofiber layer is 0.01 to 100 g/m².

## Patentansprüche

1. Eine Nanofaser-Laminatbahn, umfassend eine Nanofaserschicht, welche eine kosmetische Komponente oder eine medizinische Komponent enthält, und eine wasserlösliche Basisschicht, welche sich an mindestens einer Seite der Nanofaserschicht befindet, wobei die wasserlösliche Basisschicht weniger wasserlöslich ist als die Nanofaserschicht, wobei der Ausdruck "wasserlöslich" eine Löslichkeit angibt, dass, wenn eine Probe, welche 1 g wiegt, für 24 Stunden bei 23°C und unter atmosphärischem Druck in 10 g ionenausgetauschtem Wasser eingetaucht wird, sich mindestens 0,5 g der eingetauchten Probe in dem ionenausgetauschten Wasser auflösen.

2. Die Nanofaser-Laminatbahn gemäß Anspruch 1, wobei die Nanofaser aus einem Polymer hergestellt wird, welches wasserlöslich ist.

3. Die Nanofaser-Laminatbahn gemäß Anspruch 1 oder 2, wobei die wasserlösliche Basisschicht und die Nanofaser dasselbe Material umfassen,
wobei die wasserlösliche Basisschicht eine geringere spezifische Oberfläche aufweist als die Nanofaserschicht und,
wobei die wasserlösliche Basisschicht eine porenfreie Folie, eine poröse Folie oder ein Netz umfasst.

4. Die Nanofaser-Laminatbahn gemäß einem der Ansprüche 1 bis 3, wobei die wasserlösliche Basisschicht eine Folie ist, die aus einem Polysaccharidmaterial, Polyvinylalkohol, Agar oder Carrageenan hergestellt wird.

5. Die Nanofaser-Laminatbahn gemäß einem der Ansprüche 1 bis 4, wobei der Gehalt der kosmetischen Komponente oder der medizinischen Komponente in der Nanofaser im Bereich von 0,01 bis 70 Massen-% liegt.

6. Die Nanofaser-Laminatbahn gemäß einem der Ansprüche 1 bis 5, wobei die wasserlösliche Basisschicht aus einem Nanofaserbereich und einem folienähnlichen Bereich aufgebaut ist, wobei der Nanofaserbereich und der folienähnliche Bereich miteinander vermischt sind.

7. Die Nanofaser-Laminatbahn gemäß einem der Ansprüche 1 bis 6, wobei die wasserlösliche Basisschicht eine Schicht aus Nanofasern ist, in welcher Nanofasern miteinander zusammenhängen, um die folienähnlichen Bereiche an ihren Schnittpunkten zu bilden.

8. Die Nanofaser-Laminatbahn gemäß Anspruch 6 oder 7, wobei das Flächenverhältnis der Nanofaserbereiche zu den folienähnlichen Bereichen in einer Draufsicht 90:10 bis 10:90 beträgt.

9. Die Nanofaser-Laminatbahn gemäß einem der Ansprüche 1 bis 8, wobei die Zusammensetzung der Bahn graduell von der Zusammensetzung der wasserlöslichen Basisschicht zu der der Nanofaserschicht in ihrer Dickenrichtung variiert.

10. Die Nanofaser-Laminatbahn gemäß einem der Ansprüche 1 bis 8, welche eine klare Grenzung zwischen der Zusammensetzung der wasserlöslichen Basisschicht und der der Nanofaserschicht entlang der Dickenrichtung aufweist.

11. Die Nanofaser-Laminatbahn gemäß einem der Ansprüche 1 bis 10, welche ein flüchtiges funktionelles Mittel mit einem Dampfdruck von 13,3 Pa oder weniger bei 20°C enthält.

12. Die Nanofaser-Laminatbahn gemäß Anspruch 11, wobei das flüchtige funktionelle Mittel mindestens ein Mitglied, ausgewählt aus der Gruppe bestehend aus einem Duftstoff, einem Weißmacher und einem Geschmackskorrigens, ist.

13. Die Nanofaser-Laminatbahn gemäß Anspruch 11 oder 12, wobei der Gehalt des flüchtigen funktionellen Mittels 0,001 bis 30 Massen-% beträgt.

14. Die Nanofaser-Laminatbahn gemäß einem der Ansprüche 1 bis 13, die als Bahn, welche an die menschliche Haut, den Zahn oder das Zahnfleisch angebracht werden soll, verwendet werden soll und
wobei die Dicke der Nanofaserschicht 50 nm bis 1 mm beträgt.

15. Die Nanofaser-Laminatbahn gemäß einem der Ansprüche 1 bis 14, die als Bahn, welche an die menschliche Haut, den Zahn oder das Zahnfleisch angebracht werden soll, verwendet werden soll und
wobei das Basisgewicht der Nanofaserschicht 0,01 bis 100 g/m² beträgt.

## Revendications

1. Feuille stratifiée en nanofibres comprenant une couche de nanofibres contenant un composant cosmétique ou un composant médicinal, et une couche de base soluble dans l'eau positionnée sur au moins un côté de la couche de nanofibres, la couche de base soluble dans l'eau étant moins soluble dans l'eau que la couche de nanofibres, le terme « soluble dans l'eau » signifiant une solubilité telle que lorsqu'un échantillon qui pèse 1 g est immergé dans 10 g d'une eau échangée d'ions pendant 24 heures à 23 °C et sous pression atmosphérique, au moins 0,5 g de l'échantillon immergé est dissous dans l'eau échangée d'ions.

2. Feuille stratifiée en nanofibres selon la revendication 1, dans laquelle la nanofibre est préparée à partir d'un polymère qui est soluble dans l'eau.

3. Feuille stratifiée en nanofibres selon la revendication 1 ou 2, dans laquelle la couche de base soluble dans l'eau et la nanofibre comprennent le même matériau,
la couche de base soluble dans l'eau ayant une aire de surface spécifique plus petite que la couche de nanofibres,et
la couche de base soluble dans l'eau comprend un film sans pores, un film poreux ou une maille.

4. Feuille stratifiée en nanofibres selon l'une quelconque des revendications 1 à 3, dans laquelle la couche de base soluble dans l'eau est un film préparé à partir d'un matériau polysaccharidique, d'un alcool polyvinylique, d'agar ou de carraghénane.

5. Feuille stratifiée en nanofibres selon l'une quelconque des revendications 1 à 4, dans laquelle la teneur en composant cosmétique ou en composant médicinal dans la nanofibre est comprise dans la plage allant de 0,01 % à 70 % en masse.

6. Feuille stratifiée en nanofibres selon l'une quelconque des revendications 1 à 5, dans laquelle la couche de base soluble dans l'eau est faite d'une partie nanofibreuse et d'une partie pelliculaire, la partie nanofibreuse et la partie pelliculaire se mélangeant l'une avec l'autre.

7. Feuille stratifiée en nanofibres selon l'une quelconque des revendications 1 à 6, dans laquelle la couche de base soluble dans l'eau est une couche de nanofibres dans laquelle les nanofibres adhèrent les unes aux autres pour former les parties pelliculaires au niveau de leurs intersections.

8. Feuille stratifiée en nanofibres selon la revendication 6 ou 7, dans laquelle le rapport d'aire des parties nanofibreuses aux parties pelliculaires dans une vue de plan est de 90:10 à 10:90.

9. Feuille stratifiée en nanofibres selon l'une quelconque des revendications 1 à 8, ayant sa composition qui varie progressivement de la composition de la couche de base soluble dans l'eau à celle de la couche de nanofibres dans la direction de son épaisseur.

10. Feuille stratifiée en nanofibres selon l'une quelconque des revendications 1 à 8, ayant une limite bien définie entre la composition de la couche de base soluble dans l'eau et celle de la couche de nanofibres dans la direction de son épaisseur.

11. Feuille stratifiée en nanofibres selon l'une quelconque des revendications 1 à 10, contenant un agent fonctionnel volatil ayant une pression de vapeur inférieure ou égale à 13,3 Pa à 20 °C.

12. Feuille stratifiée en nanofibres selon la revendication 11, dans laquelle l'agent fonctionnel volatil est au moins un élément choisi dans le groupe constitué par une fragrance, un agent blanchissant et un correcteur de goût.

13. Feuille stratifiée en nanofibres selon la revendication 11 ou 12, dans laquelle la teneur en agent fonctionnel volatil est de 0,001 % à 30 % en masse.

14. Feuille stratifiée en nanofibres selon l'une quelconque des revendications 1 à 13, qui est destinée à être utilisée en tant que feuille qui est à attacher à de la peau, une dent ou une gencive humaine, et dans laquelle l'épaisseur de la couche de nanofibres est de 50 nm à 1 mm.

15. Feuille stratifiée en nanofibres selon l'une quelconque des revendications 1 à 14, qui est destinée à être utilisée en tant que feuille qui est à attacher à de la peau, une dent ou une gencive humaine, et dans laquelle le grammage de la couche de nanofibres est de 0,01 à 100 g/m².
